# EUROPEAN PATENT APPLICATION

(11) **EP 3 804 768 A1**
(43) Date of publication of application: **14.04.2021**
(21) Application number: 19811092.6
(22) Date of filing: 30.05.2019
(51) Int. Cl.: A61L 27/06, A61C 8/00, A61F 2/34, A61F 2/36, A61L 27/40

(54) **COMPOSITE MATERIAL AND BIOIMPLANT**

(30) Priority: 01.06.2018 JP 2018106267
(71) Applicant: Kyocera Corporation, Kyoto-shi, Kyoto 612-8501 (JP)
(72) Inventor: SAIGA, Kenichi, Kyoto-shi, Kyoto 612-8501 (JP); WATANABE, Kenichi, Kyoto-shi, Kyoto 612-8501 (JP); KYOMOTO, Masayuki, Kyoto-shi, Kyoto 612-8501 (JP)
(74) Representative: Viering, Jentschura & Partner mbB Patent- und Rechtsanwälte
(86) International application number: PCT/JP2019/021484
(87) International publication number: WO 2019/230871

(57) **Abstract**

A composite material in one of embodiments includes a crystal phase of titanium fluoride and a metal crystal phase of titanium. The crystal phase of the titanium fluoride is present in a first region located away from a surface in a depth direction.

## Description

### TECHNICAL FIELD

The present disclosure relates to a composite material and a bioimplant.

### BACKGROUND ART

A metal material whose surface is subjected to fluorine ion implantation has been known (for example, refer to Patent Document 1 and Non-patent Document 1).

### RELATED ART DOCUMENTS

### PATENT DOCUMENT

Patent Document 1: Japanese Patent No. 4568396

### NON-PATENT DOCUMENT

Non-patent Document 1: M. Yoshinari, Y. Oda, T. Kato, K. Okuda, "Influence of surface modifications to titanium on antibacterial activity in vitro," Biomaterials, 2001, 22, p. 2043-2048

### SUMMARY

A composite material in one of embodiments includes a crystal phase of titanium fluoride and a metal crystal phase of titanium. The crystal phase of the titanium fluoride is present in a first region located away from a surface in a depth direction.

A bioimplant in one of embodiments includes the composite material in one of the embodiments.

### BRIEF DESCRIPTION OF THE DRAWINGS

FIG. 1 is a schematic diagram illustrating a composite material in one of embodiments;
FIG. 2 is one embodiment of a bioimplant in one of embodiments;
FIG. 3 is a graph illustrating fluorine concentration measurement results in Examples; and
FIG. 4 is a graph illustrating hardness measurement results in Examples and Comparative Example.

### EMBODIMENTS

### <Composite Material>

A composite material in one of embodiments is described in detail below with reference to the drawings. For the sake of description, the drawings referred to in the following illustrate, in simplified form, only main configurations necessary for describing the embodiments. The composite material in the embodiment is therefore capable of including any arbitrary configuration not illustrated in the drawings referred to. Dimensions of the configurations in the drawings faithfully represent neither dimensions of actual configurations nor dimensional ratios of these configurations. These points are also true for a bioimplant described later.

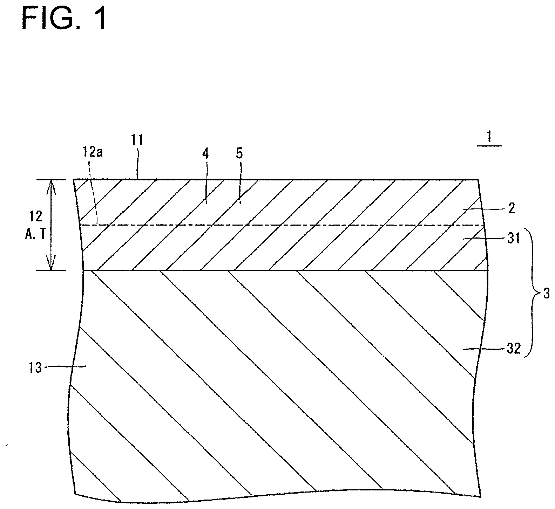

FIG. 1 is a schematic diagram illustrating the composite material in the embodiment. A cross section of a part including a surface of the composite material is enlargedly illustrated in FIG. 1.

The composite material 1 includes titanium (Ti) and fluorine (F) and includes a crystal phase 2 of titanium fluoride (hereinafter also referred to as "the crystal phase 2") and a metal crystal phase 3 of titanium (hereinafter also referred to as "the metal crystal phase 3"). The titanium fluoride that is a compound of titanium and fluorine is present in a crystal state in the crystal phase 2. The titanium is present in a state of crystal formed by metallic bonding in the metal crystal phase 3.

The composite material 1 includes fluorine and the crystal phase 2 as described above. Therefore, it becomes possible to offer antibacterial activity owing to the fluorine. Because the composite material 1 has high hardness, it becomes possible to offer excellent wear resistance or the like. The reason why the composite material 1 has the high hardness seems to be as follows.

The bonding between titanium and fluorine in the titanium fluoride is covalent bonding. The crystal phase 2 therefore serves as an obstacle to dislocation for moving the metal crystal phase 3. Accordingly, if the composite material 1 includes the crystal phase 2, an amount of energy necessary for dislocation movement becomes larger, thus leading to enhanced hardness of the composite material 1. A ratio of the crystal phase 2 tends to increase with increasing a fluorine concentration in the composite material 1.

Examples of the titanium fluoride include TiF (titanium monofluoride), TiF₂ (titanium difluoride), TiF₃ (titanium trifluoride), TiF₄ (titanium tetrafluoride), TiOF (titanium oxyfluoride), TiOF₂ (titanium oxydifluoride) and F-TiO₂ (fluorine doped titanium oxide). The titanium fluoride may be TiOF₂. The crystal phase 2 may include Ti-F-Ti bonding (covalent bonding).

As a method for measuring a crystal structure, there are, for example, Transmission Electron Microscope (hereinafter also referred to as "TEM"), X-ray Diffraction (hereinafter also referred to as "XRD") and X-ray Photoelectron Spectroscopy (hereinafter also referred to as "XPS").

The composite material 1 may include a region (first region) 12 which includes a surface 11 of the composite material 1 and has a predetermined thickness in a depth direction from the surface 11. The first region 12 is a composite phase of titanium and fluorine. A fluorine concentration of the first region 12 may be 1 ppm or more.

A thickness T of the first region 12 is, for example, 30-800 nm. If a numerical value range is indicated using "-", numerical values of a lower limit and an upper limit are included unless otherwise noted. For example, a numerical value range of 30-800 nm denotes that the lower limit is 30 nm or more and the upper limit is 800 nm or less.

The crystal phase 2 may be located in the first region 12. If satisfying this configuration, the crystal phase 2 is located in the vicinity of the surface 11. This contributes to enhancing antibacterial activity owing to the fluorine in the titanium fluoride and also increasing hardness of the surface 11 and neighborhoods thereof.

The crystal phase 2 may be located in a region with a depth range of 20-200 nm from the surface 11. The depth may be determined on the basis of the surface 11.

The metal crystal phase 3 may include a first phase 31 containing fluorine (fluorine-containing phase). In other words, the metal crystal phase 3 may include the first phase 31 including fluorine in a crystal lattice of titanium. The fluorine may be introduced as an interstitial element into the crystal lattice of titanium in the first phase 31. If the metal crystal phase 3 includes the first phase 31, the hardness of the composite material 1 can be further enhanced. The reason for this seems to be as follows.

A fluorine atom enters a space in the crystal lattice of titanium formed by metallic bonding in the first phase 31. The crystal of titanium is therefore subjected to lattice distortion according to a size of the fluorine atom thus entered. Deformation of the crystal lattice of titanium is caused by dislocation movement that is a defect of the crystal lattice. When the titanium crystal lattice strain occurred by fluorine doping, dislocation mobility is decreased and hardness of the composite material 1 is increased. Hence, with the metal crystal phase 3 including the first phase 31, the first phase 31 besides the crystal phase 2 also contributes to the hardness of the composite material 1, thereby further enhancing the hardness of the composite material 1. A ratio of the first phase 31 tends to increase with decreasing the fluorine concentration in the composite material 1.

The first phase 31 may be located in the first region 12. If satisfying this configuration, the hardness of the surface 11 and neighborhoods thereof can be enhanced because the first phase 31 is located in the vicinity of the surface 11. The first region 12 in which the first phase 31 is located is identical with the first region 12 in which the crystal phase 2 is located. This is also true for the first region 12 in which a second phase 32 described later is located, the first region 12 at which a maximum value of fluorine concentration is located, and the first region 12 at which a maximum value of hardness is located. That is, the first regions 12 in the description of the individual configurations are identical to each other.

The metal crystal phase 3 may further include a second phase 32 including no fluorine (non-fluorine containing phase) located more inside than the first phase 31. If satisfying this configuration, a region including the first phase 31, which is located closer to the surface 11 than the second phase 32, is less susceptible to damage. Specifically, because the second phase 32 includes no fluorine, the second phase 32 has higher toughness than the first phase 31. Accordingly, upon impact on the surface 11, the impact can be relaxed by the second phase 32 having relatively high toughness. Consequently, the region including the first phase 31, which is located closer to the surface 11 than the second phase 32, becomes less susceptible to damage.

The phrase that "the second phase 32 is located more inside than the first phase 31" denotes that the second phase 32 is located more away from the surface 11 than the first phase 31. The term "inside" denotes being located inside the composite material 1 relative to the surface 11. In other words, the term "inside" denotes a depth increasing direction in the composite material 1. The term "including no fluorine" denotes a state of including substantially no fluorine and being substantially free from influence of fluorine. Specifically, if the fluorine concentration is less than 1 ppm, a determination may be made that it is free from fluorine.

The second phase 32 may be located more inside than the first region 12. If satisfying this configuration, the first region 12 located closer to the surface 11 than the second phase 32 is less susceptible to damage because the second phase 32 has relatively high toughness.

The composite material 1 may further include a region (second region) 13 located more inside than the first region 12. The second region may the region including titanium but not including fluorine. The second phase 32 may be located in the second region. The second region 13 may be in contact with the first region 12. That is, the first region 12 and the second region 13 may be continuous regions in the composite material 1.

The metal crystal phase 3 may include, for example, a titanium-based metal. Examples of the titanium-based metal include pure titanium and titanium alloys. Examples of the pure titanium include industrial pure titanium, such as C.P. two types titanium whose base phase is titanium. The titanium alloys are alloys whose base phase is titanium. Examples thereof include Ti-6Al(aluminum)-4V(vanadium), Ti-15Mo(molybdenum)-5Zr(zirconium)-3Al, Ti-Nb(niobium), Ti-6Al-7Nb, Ti-6Al-2Nb-1Ta(tantalum), Ti-30Zr-Mo, Ni(nickel)-Ti, Ti-3Al-2.5v, Ti-10V-2Fe(iron)-3Al and Ti-15V-3Cr(chrome)-3Al-3Sn(tin).

The composite material 1 may further include an amorphous phase 4 including titanium and fluorine. If satisfying this configuration, the composite material 1 is less susceptible to damage because the amorphous phase 4 has high toughness.

The amorphous phase 4 may be located in the first region 12. If satisfying this configuration, the first region 12 becomes less susceptible to damage because the amorphous phase 4 has the high toughness.

The composite material 1 may further include a mixed phase 5 including the amorphous phase 4, the crystal phase 2 and the metal crystal phase 3 (the first phase 31). The mixed phase 5 is located in the first region 12 in one of the embodiments. The amorphous phases 4, the crystal phases 2 and the metal crystal phases 3 are mixed in the mixed phase 5. Although material characteristics of the individual phases are different from each other, the material characteristics of the composite material 1 in the first region 12 become characteristics according to a ratio of the individual phases. Specifically, their respective material characteristics become those obtained by averaging the material characteristics of the included individual phases, or alternatively, become characteristics close to average characteristics. That is, the composite material 1 includes the individual phases as the mixed phase 5, making it possible to reduce parts different in material characteristics in the first region. With the composite material 1 including the mixed phase 5, it is possible to reduce the probability that a material partially separates from the first region 12. This leads to improved stability of the composite material 1.

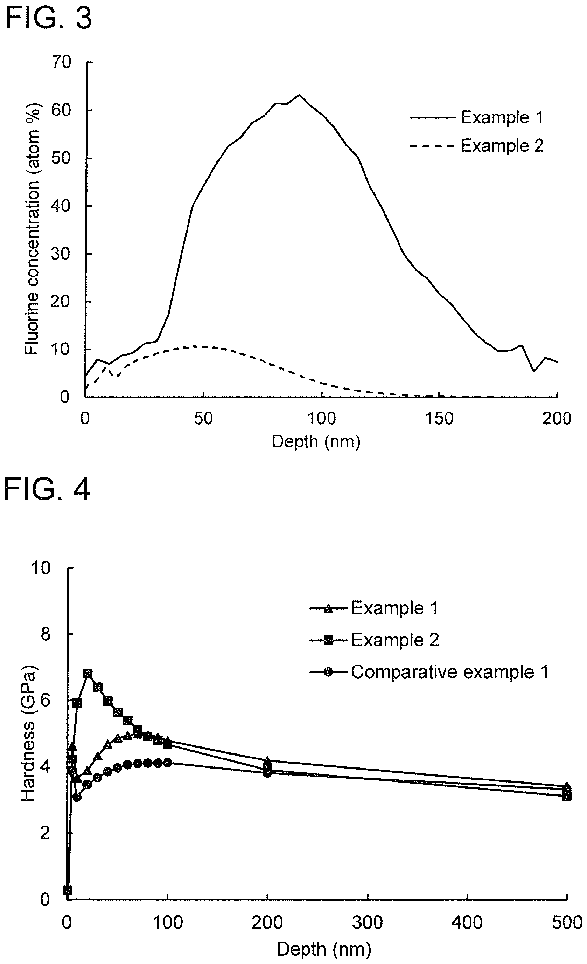

The fluorine concentration in the composite material 1 may reach a maximum value at a portion located more inside than the surface 11 (refer to FIG. 3). If satisfying this configuration, in a situation where the surface 11 is newly exposed due to wear or the like, the surface 11 having a relatively high fluorine concentration tends to be exposed. This facilitates to offer antibacterial activity over a long period of time.

The fluorine concentration may increase to a maximum value as going from the surface 11 toward the inside (refer to FIG. 3). In other words, the fluorine concentration may increase to the maximum value with increasing depth. If satisfying this configuration, in the situation where the surface 11 is newly exposed due to wear or the like, the surface 11 having the relatively high fluorine concentration tends to be exposed. This facilitates to offer antibacterial activity over the long period of time. A period of time during which the composite material 1 offers the antibacterial activity can be controlled by controlling a distribution of the fluorine concentration.

The maximum value of the fluorine concentration may be located in the first region 12. If satisfying this configuration, the maximum value of the fluorine concentration is located in the vicinity of the surface 11, thus enhancing the antibacterial activity.

The maximum value of the fluorine concentration may be located closer to a side of the surface 11 than a midportion 12a in a thickness direction A of the first region 12 (refer to FIGs. 1 and 3). If satisfying this configuration, the maximum value of the fluorine concentration is located in the vicinity of the surface 11, thus leading to the enhanced antibacterial activity.

A concentration in the fluorine concentration is an atomic concentration. The fluorine concentration in one of the embodiments is the number of fluorine atoms per unit volume relative to a sum of an ideal atomic number of titanium atoms per unit volume and the number of fluorine atoms. Examples of a method for measuring a fluorine concentration include Secondary Ion Mass Spectrometry (hereinafter also referred to as "SIMS") and XPS. The SIMS is suitable in cases where the fluorine concentration is relatively low. The XPS is suitable in cases where the fluorine concentration is relatively high.

The maximum value of fluorine concentration is, for example, 10-80 atom%. A fluorine concentration in a region from the surface 11 to less than 5 nm depth is, for example, 0.5-20 atom%. A fluorine concentration in a region with a depth range of not less than 5 nm but less than 20 nm is, for example, 2-30 atom%. A fluorine concentration in a region with a depth range of not less than 20 nm but less than 50 nm is, for example, 5-80 atom%. A fluorine concentration in a region with a depth range of not less than 50 nm but not more than 100 nm is, for example, 2-80 atom%.

The hardness of the composite material 1 may reach a maximum value at a portion located more inside than the surface 11 (refer to FIG. 4). If satisfying this configuration, in the situation where the surface 11 is newly exposed due to wear or the like, the surface 11 having relatively high hardness tends to be exposed. This enhances the possibility that the surface 11 has the high hardness over a long period of time. The surface 11 in the description of the hardness is identical to the surface 11 in the above description of the fluorine concentration.

The hardness may increase to a maximum value as going from the surface 11 toward the inside (refer to FIG. 4). In other words, the hardness may increase to the maximum value with increasing depth. If satisfying this configuration, in the situation where the surface 11 is newly exposed due to wear or the like, the surface 11 having the relatively high hardness tends to be exposed. This leads to the high hardness of the surface 11 over a long period of time.

Alternatively, the hardness may increase to a maximum value as going from the surface 11 toward the inside, and thereafter may decrease as going more inside (refer to FIG. 4). In other words, the composite material 1 may be configured so that the hardness changes moderately in the inside. With this configuration, a local stress is less likely to occur than a configuration that hardness inside the composite material 1 changes sharply. The first region 12 is therefore less likely to separate.

The maximum value of the hardness may be located in the first region 12. If satisfying this configuration, the maximum value of the hardness is located in the vicinity of the surface 11, and it is therefore possible to increase the hardness of the surface 11 and neighborhoods thereof.

The maximum value of the hardness may be located closer to a side of the surface 11 than the midportion 12a in the thickness direction A of the first region 12 (refer to FIGs. 1 and 4). If satisfying this configuration, the maximum value of the hardness is located in the vicinity of the surface 11, and it is therefore possible to increase the hardness of the surface 11 and neighborhoods thereof.

The maximum value of the hardness may be located closer to the surface 11 than the maximum value of fluorine concentration (refer to FIGs. 3 and 4). If satisfying this configuration, hardness of a portion located closer to the surface 11 than a portion having the maximum value of fluorine concentration becomes relatively high. A portion located closer to the surface 11 than the portion having the maximum value of fluorine concentration is less susceptible to damage due to wear or the like, thereby offering antibacterial activity over a long period of time.

The hardness is, for example, 3-10 GPa. The maximum value of the hardness is, for example, 5-10 GPa. The hardness is indentation hardness and denotes deformation resistance of the surface 11 if subjected to deformation. The hardness is calculated from an indentation depth when an indenter is pressed against the surface 11, and power necessary therefor. As a specific method for measuring hardness, there is, for example, nanoindentation method (according to ISO 14577).

The composite material 1 may further include an oxide film (not illustrated) located on an outermost surface. In this case, the surface 11 of the composite material 1 is composed of a surface of the oxide film. A thickness of the oxide film is, for example, 2-5 nm. Examples of composition of the oxide film include TiO₂ (titanium dioxide). The oxide film may include fluorine. For example, the oxide film is formed by oxidation treatment. Examples of oxidation treatment include natural oxidation, heat treatment, oxygen plasma treatment, immersion into an oxidation solution, and anodic oxidation.

A content of titanium may be larger than a content of fluorine in the composite material 1. The composite material 1 may include titanium as a main composition. The main composition is a composition whose mass ratio is highest in the composite material 1.

### <Method for Manufacturing Composite Material>

A method for manufacturing a composite material in one of embodiments is described in detail below by exemplifying the case of obtaining the above composite material 1.

Firstly, titanium-based metal is prepared. The titanium-based metal may be washed if necessary. Washing may be carried out using, for example, an organic solvent. Examples of the organic solvent include ethanol and acetone. The organic solvents exemplified here may be used by being mixed together. The washing may be carried out while applying ultrasonic waves. The titanium-based metal after being washed may be subjected to, for example, vacuum drying in a desiccator.

Subsequently, fluorine ions are implanted into a surface of the titanium-based metal, thereby obtaining the composite material 1. Examples of implantation conditions of fluorine ion include the following conditions.
Implantation energy: more than 30 keV but not more than 80 keV
Implantation dose: 1×10¹⁶ - 5×10¹⁷ atom/cm²

The obtained composite material 1 may be washed if necessary. Conditions of the washing may be identical to those exemplified in the above titanium-based metal. The composite material 1 after being washed may be subjected to, for example, vacuum drying in the desiccator.

Although the above embodiment has illustrated and described the case of obtaining the composite material 1 by the fluorine ion implantation, the method for manufacturing the composite material 1 is not limited thereto. Other methods other than the fluorine ion implantation are employable as long as the composite material 1 is obtainable.

### <Bioimplant>

A bioimplant in one of embodiments is described in detail below with reference to the drawings. As an example of the bioimplant, a dental implant is described in the present embodiment.

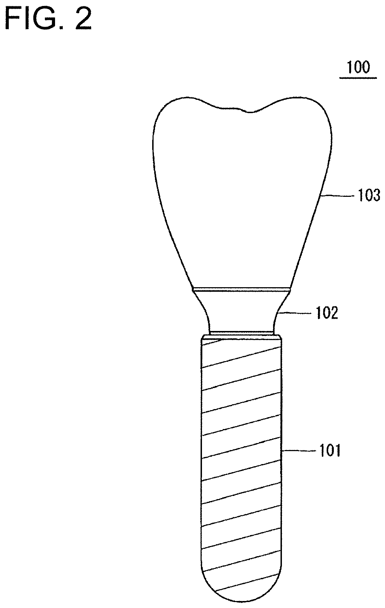

FIG. 2 is a schematic diagram illustrating an appearance of the dental implant in the embodiment.

The dental implant 100 includes a fixture 101, an abutment 102 attached to an end portion of the fixture 101, and an artificial tooth 103 attached to the fixture 101 with the abutment 102 interposed therebetween.

The fixture 101, the abutment 102 and the artificial tooth 103 in the dental implant 100 include the composite material 1. Because the composite material 1 has the antimicrobial activity and high hardness as described earlier, the dental implant 100 is capable of reducing growth of bacteria and offering excellent durability against brushing, repetitive use, washing or the like.

For example, the fixture 101, the abutment 102 and the artificial tooth 103 may be individually formed only by the composite material 1. Alternatively, a part of these may be composed by the composite material 1, and the rest may be composed by a material other than the composite material 1. Still alternatively, at least one of the fixture 101, the abutment 102 and the artificial tooth 103 may include the composite material 1, and the rest may include a material other than the composite material 1. This configuration contributes to reducing the growth of bacteria in the surface of the implant. For example, a reduction in growth of anaerobic bacteria can be expected because the fixture 101 and the abutment 102 are used in an oxygen-deficient atmosphere. For example, a reduction in growth of facultative anaerobic bacteria and aerobic bacteria can be expected because the artificial tooth 103 is exposed in a buccal cavity and exposed to air. Thus, the composite material 1 may suitably be applied to the fixture 101, the abutment 102 and the artificial tooth 103 according to the kind of bacteria whose growth needs to be reduced, and necessary antimicrobial performance.

The first region 12 in the composite material 1 may be located at, for example, a portion of the dental implant 100 with which bacteria is likely to contact and which is likely to wear out. For example, the dental implant 100 may be configured so that the first region 12 is located at individual surfaces of the fixture 101, the abutment 102 and the artificial tooth 103. Alternatively, the dental implant 100 may be configured so that the first region 12 is located at individual connection parts of the fixture 101, the abutment 102 and the artificial tooth 103. This is also true for other bioimplant described later and members other than the bioimplant.

While the embodiments in the present disclosure has been illustrated and described above, it is to be understood that the present disclosure is not limited to the foregoing embodiments and may be made into any arbitrary ones insofar as they do not depart from the spirit and scope of the present disclosure.

For example, even though the case where the bioimplant is the dental implant has been described as an example in the above embodiments, the bioimplant is not limited thereto. For example, the bioimplant may be an implant for biocompatible metal, such as titanium. Examples of other bioimplant include artificial joints such as femoral stems and acetabulum shells, and spine surgery implants such as spine fixation instrumentation.

Even though the case where the composite material 1 is intended for the bioimplant has been described as an example in the above embodiments, the composite material 1 is not limited to the purpose of the bioimplant. That is, the composite material 1 may be used as a material of a member for which antibacterial activity and high hardness are necessary. Examples of other members include orthodontic wire, surgical instrument, hypodermic needles, glasses frames, tableware, food factory lines, water bottle faucets, kitchen knives, toilets, Washlet (registered trademark), taps, and water and sewage pipes.

The present disclosure is described in detail below by giving examples. However, the present disclosure is not limited to the following examples.

### EXAMPLES

### [Examples 1 and 2]

### <Composite Material Manufacturing>

Firstly, the following specimen was prepared.

Specimen: 1mm thick pure titanium composed of C. P. two types titanium

The above specimen was formed in a disk shape having a diameter of 14 mm and a thickness of 1 mm. This was washed with ethanol and acetone while applying ultrasonic waves, and was then subjected to vacuum drying in a desiccator. Thereafter, fluorine ions were implanted into a surface of the specimen under different conditions, thereby obtaining a composite material 1 of Example 1 and that of Example 2.

Implantation conditions of the fluorine ions were as follows.

### (Example 1)

Implantation energy: 40 keV
Implantation dose: 5×10 atom/cm²

### (Example 2)

Implantation energy: 40 keV
Implantation dose: 5×10¹⁶ atom/cm²

Evaluations were made of the obtained composite materials 1 after being washed with ethanol and acetone while applying ultrasonic waves, followed by vacuum drying in the desiccator.

### [Comparative Example 1]

Comparative Example 1 was the same specimen as Examples 1 and 2, except that no fluorine ions were implanted therein.

### <Evaluation>

Measurements were made of fluorine concentration, hardness and crystal structure of the composite materials 1 of Examples 1 and 2. Measurement was also made of antimicrobial activity of the composite material 1 of Example 1. Measurements were made of hardness and antimicrobial activity of Comparative Example 1.

FIG. 3 is a graph illustrating measurement results of fluorine concentration in Examples 1 and 2.

### (Fluorine Concentration)

The fluorine concentrations of Examples 1 and 2 were measured by the XPS and the SIMS. Specifically, a region where the fluorine concentration is relatively high and goes beyond a measurement range of the SIMS was subjected to fluorine concentration calculation by the XPS, and regions other than the above region was subjected to fluorine concentration calculation by the SIMS. Specifically, a range where the fluorine concentration was less than 10 atom% was subjected to fluorine concentration calculation by the SIMS. A range where the fluorine concentration was 10 atom% or more was subjected to fluorine concentration calculation by the XPS. The measurement by the XPS was carried out at a depth of 0-200 nm, and the measurement by the SIMS was carried out at a depth of 0-900 nm. FIG. 3 illustrates only the measurement results at the depth of 0-200 nm. In FIG. 3, the depth 0 nm indicates the surface 11 of the composite material 1. This is also true for FIG. 4 described later. Measurement conditions for the XPS and SIMS are as follows.

### (Measurement Conditions for the XPS)

Analyzer: X-ray Photoelectron Spectroscopy Analyzer "PHI Quantera II" manufactured by ULVAC-PHI Corporation
X-ray source: Monochrome AlKα
Sputtering ion: Ar⁺
Accelerating voltage: 4 kV

### (Measurement Conditions for the SIMS)

Analyzer: Secondary Ion Mass Analyzer "D-SIMS 6650" manufactured by ULVAC-PHI Corporation
Primary ion species: Cs⁺
Secondary ion polarity: Negative
Accelerating voltage: 2 kV
Beam current: 25 nA
Charge compensation: None
Raster size: 400 µm

Measurement results showed the following. That is, a maximum value of fluorine concentration was located at a depth of 90 nm in Example 1. The maximum value of the fluorine concentration in Example 1 was 63 atom%. A maximum value of fluorine concentration was located at a depth of 46 nm in Example 2. The maximum value of the fluorine concentration in Example 2 was 11 atom%.

A region from the depth 0 nm (the surface 11) to a depth at which the fluorine concentration reached 1 ppm was a region 12, and a thickness T thereof was measured. The measurement results are as follows.

### {Thickness T of the first region}

Example 1: 740 nm
Example 2: 390 nm

FIG. 4 is a graph illustrating measurement results of hardness in Examples 1 and 2 and Comparative Example 1.

### (Hardness)

The hardness was measured by nanoindentation method (according to ISO 14577). The measurement was carried out at a depth of 0-1000 nm. FIG. 4 illustrates only the measurement results at the depth of 0-500 nm.

Measurement conditions of hardness are as follows:
Measuring device: "Nanoindenter XP" manufactured by MTS Systems Corporation
Measuring mode: Continuous stiffness measurement
Indentation depth: Maximum value 1000 nm
Hardness unit: Vickers hardness

Measurement results showed the following. That is, a maximum value of hardness was located at a depth of 70 nm in Example 1, and the maximum value of hardness in Example 1 was 5 GPa. A maximum value of hardness was located at a depth of 20 nm in Example 2, and the maximum value of hardness in Example 2 was 7 GPa.

### (Crystal Structure)

Crystal structure was evaluated by the TEM, XRD and XPS. In individual measurements of the TEX, XRD and XPS, the first region 12 was determined from the thickness T of the first region 12, and a region located more inside than the first region 12 was a second region.

Measurement conditions for the TEM are as follows.
Analyzer: Transmission electron microscope "Talos F200X" manufactured by FEI Corporation
Accelerating voltage: 200 kV
Beam current value: 150 pA
Measurement location: A cross section of the composite material 1 being cut out in a thickness direction

Measurement conditions for the XRD are as follows.
Analyzer: "X'Pert PRO-MRD" manufactured by PANalytical Corporation
Tube: CuKα
Incidence angle: 0.5°
Measurement range: 10-120°

Measurement conditions for the XPS are the same as those for the fluorine concentration.

Firstly, a cross-section observation by the TEM was carried out. A diffraction pattern was determined by referring to data base (TiOF2: ICDD No.00-008-0060, titanium α-phase: ICDD No.00-044-1294) provided by International Centre for Diffraction Data, ICDD. As a result of the observation, a diffraction pattern assigned to TiOF₂ (the crystal phase 2) was obtained in the first region 12, and a diffraction pattern of titanium α-phase (the second phase 32) was obtained in the second region in both Examples 1 and 2.

The first phase 31 was observed in the first region 12 in both Examples 1 and 2. A larger number of the crystal phase 2 than the crystal phase 2 were observed in Example 1. A larger number of the first phases 31 than the crystal phase 2 were observed in Example 2. The amorphous phase 4 and the mixed phase 5 were observed in the first region 12 in Example 1.

Then, measurement by the XRD was carried out. A diffraction pattern was determined by referring to the JCPDS provided by ICDD. As a result of the measurement, a crystal structure different from that of the second region was observed in the first region 12.

Subsequently, measurement by the XPS was carried out. Peak assignment is presented in Table 1. As a result of the measurement, peaks assigned to TiF₃, TiF₄ and F-TiO₂ were obtained in both Examples 1 and 2. Although a peak assigned to Ti-F-Ti bonding was obtained, the peak seems to be caused by a crystal of titanium fluoride. In other respect, states illustrated in FIG. 1 were confirmed.

**[Table 1]**

| | | | | | |
|---|---|---|---|---|---|
| O1s | Formula | Element | Energy (eV) | Referrence | |
| | TiO_{0.73} | O1s | 531.7 | Kuzetsov M. V. et al. | J. Electron Spectrosc. Re;at. Phenom. 58:196 (1992) |
| | F-TiO2 | O1s | 531.0 | T.Tanuma et al. | Catal Lett 136:77-82 (2010) |
| | TiO₂ | O1s | - | Pre-installed databases on XPS devices | |
| | TiO | O1s | - | Pre-installed databases on XPS devices | |
| F1s | TiF₃/TiF₄ | F1s | 684.9 | C. Mousty-Desbuquoit et al. | Inorg. Chem. 26:1212-1217 (1987) |
| | F-TiO₂ | F1s | 685.6 | T.Tanuma et al. | Catal Lett 136:77-82 (2010) |
| | Ti-F-Ti lattice | F1s | 686.8 | J. Y. Ruzicka et al. | RSC Adv. 4:20649 (2014) |
| Ti2p3/2 | F-TiO₂ | Ti2p3/2 | | T.Tanuma et al. | Catal Lett 136:77-82 (2010) |
| | TiF₃ | Ti2p3/2 | 461.2 | F Fracassi et al. | Pure & Appl. Chem. 64:703-703 (1992) |
| | TiF₄ | Ti2p3/2 | 461.6 | C. Mousty-Desbuquoit et al. | Inorg. Chem. 26:1212-1217 (1987) |
| | TiO | Ti2p3/2 | 455.1 | M Matoba et al. | J. Phvs. Soc. Jpn. 63:1429-1440 (1994) |
| | Ti | Ti2p3/2 | 453.9 | M Matoba et al. | J. Phys. Soc. Jpn. 63:1429-1440 (1994) |

### (Antibacterial Activity)

Antibacterial activity was measured by a film adhesion test using staph aureus (according to JIS Z 2801) .

Measurements results were as follows.

### Attached viable cell count (CFUs)

Example 1: <10 (detection limit or less)
Comparative Example 1: 17667

As a result of the measurement, the attached viable cell count was the detection limit or less in Example 1. Antibacterial activity value of Example 1 was 3.2. Consequently, it became apparent that Example 1 had antibacterial effect.

### DESCRIPTION OF THE REFERENCE NUMERAL

- 1: composite material
- 2: crystal phase of titanium fluoride
- 3: metal crystal phase of titanium
31 first phase
32 second phase
- 4: amorphous phase
- 5: mixed phase
- 11: surface
- 12: first region
12a midportion
T thickness
A thickness direction
- 13: second region
- 100: dental implant
- 101: fixture
- 102: abutment
- 103: artificial tooth

## Claims

1. A composite material, comprising:
a crystal phase of titanium fluoride; and
a metal crystal phase of titanium,
the crystal phase of the titanium fluoride being present in a first region located away from a surface in a depth direction.

2. The composite material according to claim 1, wherein the titanium fluoride is TiOF₂.

3. The composite material according to claim 1 or 2, wherein the metal crystal phase comprises a first phase comprising fluorine.

4. The composite material according to claim 3, wherein the first phase is located in the first region.

5. The composite material according to claim 3 or 4, wherein
the metal crystal phase further comprises a second phase located more inside than the first phase, and
the second phase comprises no fluorine.

6. The composite material according to claim 5, wherein the second phase is located more inside than the first region.

7. The composite material according to any one of claims 1 to 6, further comprising:
an amorphous phase comprising titanium and fluorine.

8. The composite material according to claim 7, further comprising:
a mixed phase comprising the amorphous phase, the crystal phase of the titanium fluoride, and the metal crystal phase.

9. The composite material according to any one of claims 1 to 8, wherein a fluorine concentration reaches a maximum value at a portion located more inside than the surface.

10. The composite material according to claim 9, wherein the fluorine concentration increases to the maximum value when going from the surface toward inside.

11. The composite material according to claim 9 or 10, wherein the fluorine concentration reaches the maximum value in the first region.

12. The composite material according to claim 11, wherein the fluorine concentration reaches the maximum value at a side closer to the surface than a midportion in a depth direction of the first region.

13. The composite material according to any one of claims 1 to 12, wherein hardness reaches a maximum value at a portion located more inside than the surface.

14. The composite material according to claim 13, wherein the hardness increases to the maximum value when going from the surface toward inside.

15. The composite material according to claim 13 or 14, wherein the hardness reaches the maximum value in the first region.

16. The composite material according to claim 15, wherein the hardness reaches the maximum value at a side closer to the surface than a midportion in a depth direction of the first region.

17. The composite material according to any one of claims 1 to 16, wherein hardness reaches a maximum value at a side closer to the surface than a position at which a fluorine concentration reaches a maximum value.

18. The composite material according to any one of claims 1 to 17, the composite material being intended for a bioimplant.

19. A bioimplant, comprising:
the composite material according to any one of claims 1 to 18.
